Europäisches Patentamt

European Patent Office

Office européen des brevets

(11) Publication number: **0 068 744**
**B1**

# EUROPEAN PATENT SPECIFICATION

(45) Date of publication of patent specification: 03.12.86

(21) Application number: 82303167.9

(22) Date of filing: 17.06.82

(51) Int. Cl.⁴: **A 61 M 1/00**, A 61 F 5/44,
A 61 G 9/00, F 16 K 21/00

(54) Tap for drainage bag.

(30) Priority: 30.06.81 GB 8120082
18.12.81 GB 8138193

(43) Date of publication of application:
05.01.83 Bulletin 83/01

(45) Publication of the grant of the patent:
03.12.86 Bulletin 86/49

(84) Designated Contracting States:
AT BE CH DE FR IT LI LU NL SE

(56) References cited:
CH-A- 398 225
GB-A- 920 618
GB-A-2 061 466
GB-A-2 080 247
US-A-3 952 727
US-A-4 055 179
US-A-4 333 480

(73) Proprietor: CRAIG MEDICAL PRODUCTS
LIMITED
Claygate House 46 Albert Road North
Reigate Surrey RH2 9EN (GB)

(72) Inventor: Steer, Peter L.
Malt House Works Station Road
East Grinstead Sussex (GB)
Inventor: Edwards, John V.
5 Lodge Close
East Grinstead Sussex (GB)

(74) Representative: Cook, Anthony John et al
D. YOUNG & CO. 10, Staple Inn
London, WC1V 7RD (GB)

Courier Press, Leamington Spa, England.

## Description

The present invention relates to a tap for a drainage bag.

Various types of taps for drainage bags have heretofore been known. Examples of taps fixed to liquid containers are shown in Swiss Patent No. 398225 and U.K. Published Application No. 2 061 466.

The present invention aims to provide a tap which can be readily fixed in a face-to-face manner to a wall of a plastic drainage bag, and which is easy to operate.

According to the present invention there is provided a tap for fixing to a wall of a plastics drainage bag comprising:

(a) a body with a substantially cylindrical recess formed therein, and

(b) a substantially cylindrical valve member having a handle positioned thereon, said valve member being held within said recess in said body, said valve member being insertable and rotatable in said cylindrical recess of said body, the body having a flange thereon, whereby said body may be secured to a wall of said drainage bag in a face-to-face manner; characterised in that the body has a rib located on the exterior surface thereof, and in that the handle is deformable and has a detent surface thereon which can be engaged with the rib so as to both hold the valve member within the recess when the handle is undeformed and permit its withdrawal therefrom in a direction axially of the recess when the handle is deformed, the handle being arranged for rotating the valve member relative to the body between valve-opened and valve-closed positions.

A tap for a drainage bag is particularly disclosed herein which includes two interengageable parts. The first part is a body having a substantially cylindrical recess. The first part includes a flange, whereby the body may be secured to a wall of the bag. The second part is a valve member which is insertable and rotatable in the cylindrical recess of the body. The valve member is in the form of a substantially hollow tube having a port. At one rotational position of the valve member relative to the body, the port in the valve member registers with a port in the body, thereby opening the valve. The valve member has a handle portion which extends substantially radially therefrom. The handle has a deformable detent engageable with a rib on the body. When the detent on the handle engages the rib on the body, as in normal use, the valve member is held within the body. Yet the valve member is not prevented from rotating relative to the body.

According to a preferred feature of the invention, the handle is in the form of a flat blade secured to or integral with an arm projecting radially outwardly from the valve member. The handle may have one side marked or coded (e.g., by color or other indicia) to indicate that the valve is "closed". The other side is marked or coded to indicate that the valve is "open". The body is preferably integral with or secured to a flat flange of plastic material. Such a flange is readily heat-welded, or adhesively fixed, in a face-to-face manner, to a wall of the bag, and such fixing is particularly well suited to rapid mass-production methods of manufacture.

The body and the valve member can be readily separated when it is desired to clean or sterilize the tap.

FIG. 1 is a front view of a drainage bag including an example of a tap according to the invention;

FIG. 2 is a front view of the body part of the tap shown in FIG. 1;

FIG. 3 is a side view of the body part shown in FIG. 2;

FIG. 4 is a side view of a valve member of the tap shown in FIG. 1;

FIG. 5 is a part view of the valve member shown in FIG. 4;

FIG. 6 is a horizontal cross-sectional view of the tap shown in FIG. 1 illustrating the valve member in the "open" position; and

FIG. 7 is a horizontal cross-sectional view of the tap shown in FIG. 1 illustrating the valve member in the "closed" position, wherein an air entry path to the outlet tube is illustrated.

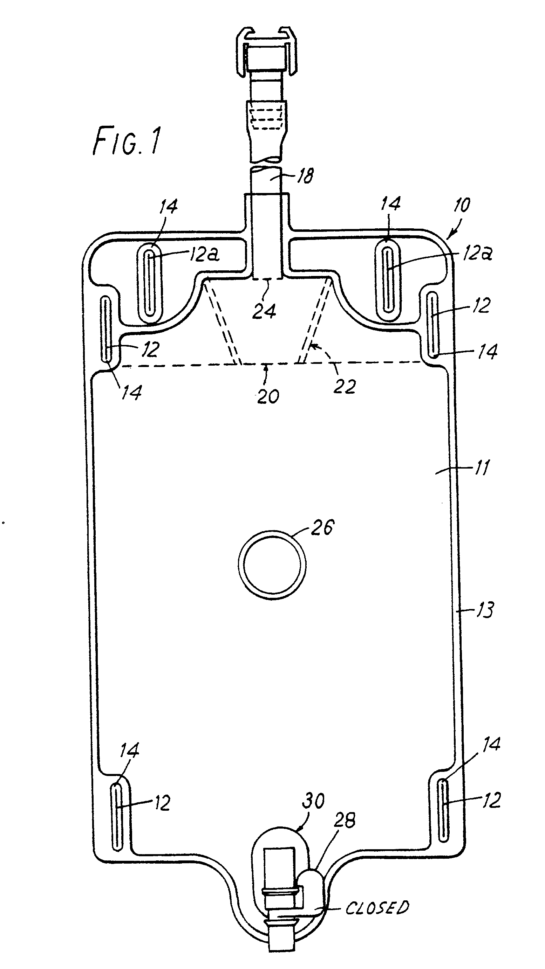

The drainage bag 10 of the present invention is illustrated in FIG. 1. The bag 10 is made from two sheets 11 of plastic which are joined together around their edges, preferably by a weld seam 13 of the "double tramline" type. A number of slits 12 are provided through the superposed pair of plastic sheets 11 which constitute the walls of the bag 10. The slits 12 are preferably surrounded by strengthening beads 14. The slits 12 are provided in order that they be able to receive straps or tapes (not shown) for fastening the bag 10 to a user's leg. There are also slits 12a to facilitate hanging the bag 10 up, if desired. The bag 10 includes an integral inlet tube 18. The upper region of the bag 10 includes a non-return or anti-reflux valve 20. The anti-reflux valve 20 is preferably made of a single piece of plastic, which in combination with one of the walls 11 of the bag 10, to which it is welded, as shown at 22, defines a liquid entry space immediately downstream of the outlet 24 of the tube 18.

In the preferred embodiment of the invention, the center portion of the walls 11 of the bag 10 are preferably welded togther using a closed-loop weld 26 which is typically of circular configuration. The weld 26 prevents undue "pouching" or "bulging" of the bag 10 as the bag 10 fills with liquid.

While the bag 10 is generally rectangular, its lower region has a downwardly extending extension 28 to receive the tap 30 which is welded or otherwise secured thereto.

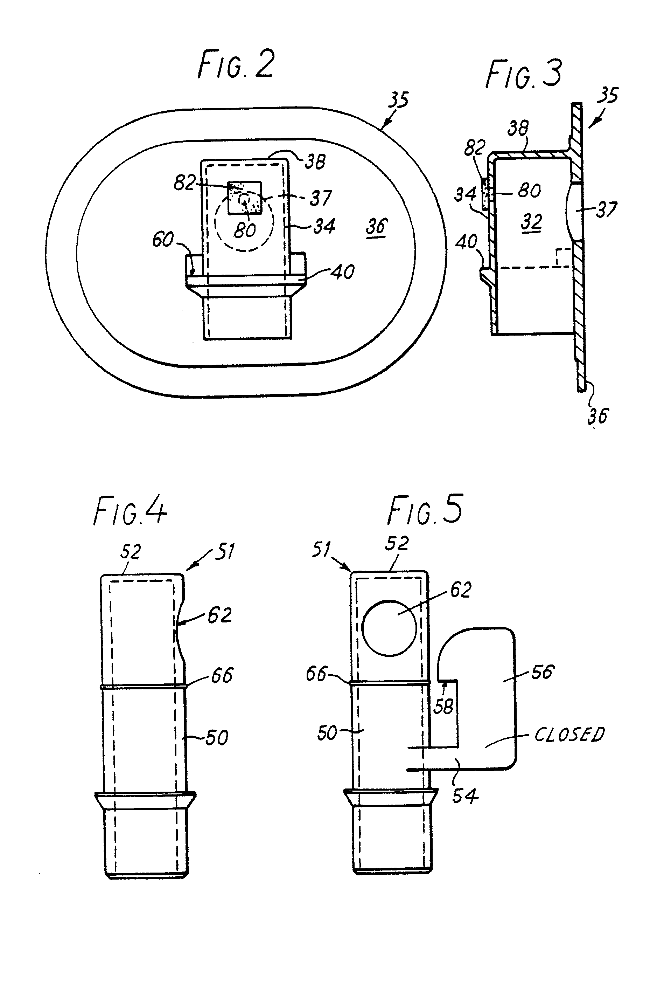

The tap 30 is comprised of a body 35, shown in FIGS. 2 and 3, and a valve member 51, shown in FIGS. 4 and 5. The valve body 35 is preferably made of plastic, and it is typically injection molded. The valve body 35 includes a generally cylindrical recess 32, and the substantially cylindrical wall 34 of the recess 32 is attached to, or integral with, a flat flange 36. The recess 32 is closed by a top wall 38. An external rib 40 is integral with the wall 34 and merges into the flange 36. A liquid exit port 37 extends through the flange 36 and opens into the recess 32. When the valve body 35 is secured to the wall 11 of the bag 10, the liquid exit port 37 is aligned with a hole or aperture (not shown) which is pre-punched in the wall 11 of the bag 10. Thus, a liquid communication path extends from the interior of the bag 10 to the recess 32.

The valve member 51, shown in FIGS. 4 and 5 may also be injection molded from plastic. The valve member 51 includes a substantially tubular hollow housing 50 closed at one end by a wall 52. The valve member 51 has an arm 54 which extends radially outwardly therefrom. A flat handle 56 is formed as an integral part of the arm 54 and extends upwardly therefrom. The handle 56 serves as a deformable portion of the valve member 51. The handle 56 has a detent surface 58 positioned for engagement with a coopering detent surface 60 on the rib 40 of the valve body 35. As illustrated in FIG. 5, one surface of the handle 56 bears the legend "closed" and the other (non-visible) surface bears the legend "open". A liquid exit port 62 is provided in the housing 50. The liquid exit port 62 registers with the liquid exit port 37 when the valve member 51 is in one rotational position relative to the valve body 35. The liquid exit port 62 is closed off by the valve body 35 when the valve member 51 is in the other rotational position relative to the valve body 35. In normal use, the handle 56 is moved between its two possible limit positions. At one limit position, the handle 56 is generally parallel to the flange 36 on one side of the central axis of the valve body 35. In the other position, the handle 56 is generally parallel to the flange 36 on the other side of the central axis of the valve body 35. When the ports 37, 62 are aligned the "open" legend is exposed, whereas when the handle 56 and arm 54 are positioned as shown in FIG. 5 the ports 37, 62 are not aligned, so the "closed" legend is exposed. The apparatus of the present invention is particularly convenient to use and is easy to operate, even by old and infirm people. In addition, the clear legend on the handle 56 avoids any confusion as to whether the tap 30 is closed or open.

If it is desirable to separate the valve member 51 from the valve body 35, for cleaning, for example, then the handle 56 is deformed away from the housing 50 so that the detent surfaces 58, 60 are disengaged. When disengaged, the valve member 51 is readily withdrawn axially downwards.

A land 66 of short axial length is preferably provided on the external cylindrical surface of the valve member 51, in order to improve the retention of the valve member 51 in the valve body 35.

With reference now to FIGS. 1, 2, 6, and 7, the cylindrical wall 34 has a hole 80 formed therethrough. The hole 80 is covered by a pad 82 of air-permeable material.

If desired, the air-permeable pad 82 may be impregnated with a bacteriacide or an odor removing substance.

The pad 82 is attached to the cylindrical wall 34 in any desired manner. In the preferred embodiment of the invention, an adhesive material is used to attach the pad 82 to the body 34.

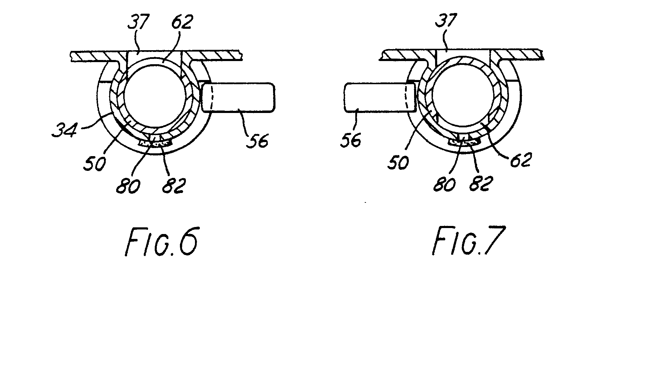

As shown in FIG. 7, the hole 80 is located so that it is aligned with the hole 62 when the handle 56 is in its closed position, and, as shown in FIG. 6, the hole 80 is shut off when the handle 56 is in its open position. When the tap is closed, air can enter the interior of the valve member 50, and can thereby enter the interior of a drainage tube extending down from the tap outlet. In this way, there is no build-up of negative pressure in the drainage tube, a condition known as "pooling". At the same time, no liquid can exit through the hole 80.

It will be seen that the invention as particularly disclosed and illustrated herein provides a simple two-part tap which can readily be mass produced, which can readily be rapidly fixed to a drainage bag, and which is simple, easy, and relatively foolproof to operate.

**Claims**

1. A tap for fixing to a wall of a plastics drainage bag comprising:

(a) a body (35) with a substantially cylindrical recess (32) formed therein, and

(b) a substantially cylindrical valve member (51) having a handle (56) positioned thereon, said valve member being held within said recess in said body, said valve member being insertable and rotatable in said cylindrical recess of said body, the body having a flange (36) thereon, whereby said body may be secured to a wall of said drainage bag in a face-to-face manner; characterised in that the body has a rib (40) located on the exterior surface thereof, and in that the handle is deformable and has a detent surface (58) thereon which can be engaged with the rib (40) so as to both hold the valve member within the recess when the handle is undeformed and permit its withdrawal therefrom in a direction axially of the recess when the handle is deformed, the handle being arranged for rotating the valve member relative to the body between valve-opened and valve-closed positions.

2. The tap of claim 1 wherein said valve member is in the form of a substantially hollow tube having a port (62), and at one rotational position of said valve member relative to said body, said port in said valve member registers with a similar port (37) in said body, thereby opening said valve.

3. The tap of Claim 2 wherein said valve body includes an air-permeable vent (80, 82) opposite said similar port in said body, whereby when said valve is open said air-permeable vent is closed, and when said valve is closed, said air-permeable vent is open.

4. The tap of Claim 3 wherein said air-permeable vent is a port (80) which is covered by an air-permeable material (82).

5. The tap of Claim 4 wherein said air-permeable material is impregnated with a bacteriacide.

6. The tap of Claim 4 wherein said air-permeable material is impregnated with an odor removing material.

7. The tap of Claim 1 wherein said handle portion (56) of said valve member extends substantially radially therefrom.

8. The tap of Claim 7 wherein said handle is in the form of a flat blade secured to or integral with an arm projecting radially outwardly from said valve member.

9. The tap of Claim 8 wherein said handle has one side marked or coded (e.g. by color or other indicia) to indicate that the valve is "closed".

10. The tap of Claim 8 wherein said handle has one side marked or coded to indicate that the valve is "open".

11. The tap of any preceding claim wherein said body is formed to be integral with a flat flange of plastic, said flange being welded in a face-to-face manner, to a wall of said bag.

12. The tap of any preceding claim in combination with a bag, wherein said body is formed to be integral with a flat flange (36) of plastic, said flange being adhesively fixed, in a face-to-face manner, to a wall of said bag (10).

## Revendications

1. Robinet destiné à être fixé à une paroi d'un sac de drainage en matière plastique, comprenant:

(a) un corps (35) dans lequel est formé un évidement (32) sensiblement cylindrique, et

(b) un élément de soupape (51) sensiblement cylindrique sur lequel est disposée une poignée (56), ledit élément de soupape étant maintenu à l'intérieur dudit évidement dans ledit corps, ledit élément de soupape pouvant être introduit et pouvant tourner dans ledit évidement cylindrique dudit corps, le corps portant une bride (36) grâce à laquelle ledit corps peut être fixé à une paroi dudit sac de drainage, face contre face; caractérisé en ce que le corps comporte une nervure (40) placée sur sa surface extérieure, et en ce que la poignée est déformable et porte une surface de détente (58) que l'on peut engager sur la nervure (40) de manière à, d'une part, maintenir l'élément de soupape à l'intérieur de l'évidement quand la poignée n'est pas déformée et, d'autre part, à permettre son retrait dans une direction axiale à l'évidement quand la poignée est déformée, la poignée étant disposée de façon à faire tourner l'élément de soupape par rapport au corps entre une position d'ouverture de soupape et une position de fermeture de soupape.

2. Robinet selon la revendication 1, caractérisé en ce que ledit élément de soupape de présente sous la forme d'un tube sensiblement creux comportant un orifice (62), et dans une position de rotation dudit élément de soupape par rapport audit corps, ledit orifice dudit élément de soupape coïncide avec un orifice similaire (37) ménagé dans ledit corps, ouvrant ainsi ladite soupape.

3. Robinet selon la revendication 2, dans lequel ledit corps de soupape comporte un évent (80, 82) perméable à l'air, en regard dudit orifice similaire ménagé dans ledit corps, pour que, quand ladite soupape est ouverte, ledit évent perméable à l'air soit fermé, et que, quand ladite soupape est fermée, ledit évent perméable à l'air soit ouvert.

4. Robinet selon la revendication 3, dans lequel ledit évent perméable à l'air est un orifice (80) qui est recouvert d'une matière (82) perméable à l'air.

5. Robinet selon la revendication 4, dans lequel ladite matière perméable à l'air est imprégnée d'un bactéricide.

6. Robinet selon la revendication 4, dans lequel ladite matière perméable à l'air est imprégnée d'une matière désodorisante.

7. Robinet selon la revendication 1, dans lequel ladite partie poignée (56) dudit élément de soupape en part sensiblement radialement.

8. Robinet selon la revendication 7, dans lequel ladite poignée se présente sous la forme d'une lame plate fixée à ou faisant corps avec un bras qui fait saillie radialement à l'extérieur dudit élément de soupape.

9. Robinet selon la revendication 8, dans lequel ladite poignée a un côté marqué ou codé (par une couleur ou d'autres repères par exemple) pour indiquer que la soupape est "fermée".

10. Robinet selon la revendication 8, dans lequel ladite poignée a un côté marqué ou codé pour indiquer que la soupape est "ouverte".

11. Robinet selon l'une quelconque des revendications précédentes, dans lequel ledit corps est formé d'une seule pièce avec une bride plate en matière plastique, ladite bride étant soudée, face contre face, à une paroi dudit sac.

12. Robinet selon l'une quelconque des revendications précédentes, en combinaison avec un sac, dans lequel ledit corps est formé d'une seule pièce avec une bride plate (36) en matière plastique, ladite bride étant fixée par adhésif, face contre face, à une paroi dudit sac (10).

## Patentansprüche

1. Hahn für Drainagesack zum Anbringen an einer Wand eines Drainagesackes aus Kunststoff, umfassend:

(a) einen Körper (35) mit einer darin ausgebildeten zylindrischen Aussparung (32), und

(b) ein im wesentlichen zylindrisches Ventilteil (51) mit einem daran angeordneten Handgriff, wobei das Ventilteil in der Aussparung im Körper gehalten wird und das Ventilteil in die zylindrische Aussparung des Körpers einsetzbar und in ihr drehbar ist, und weiter der Körper einen daran angeordneten Flansch aufweist, wodurch der Körper an einer Wand des Drainagesackes, derselben zugewandt, befestigt werden kann, dadurch gekennzeichnet, daß der Körper auf seiner Außenfläche eine Rippe (40) aufweist, und daß der Handgriff verformbar ist und eine Rastfläche (58) aufweist, die mit der Rippe (40) in Eingriff bringbar ist, um sowohl das Ventilteil in der Aussparung zu halten, wenn der Handgriff nicht verformt ist, als auch ein Herausziehen daraus in axialer Richtung der Aussparung zu gestatten, wenn der Handgriff verformt ist, wobei der Handgriff zum Drehen des Ventilteils relativ zum Körper zwischen einer geöffneten und einer geschlossenen Ventilstellung angeordnet ist.

2. Hahn nach Anspruch 1, dadurch gekennzeichnet, daß das Ventilteil die Form eines im wesentlichen hohlen Rohres mit einer Öffnung (62) aufweist, und an einer Drehstellung des Ventilteils relativ zum Körper die Öffnung im Ventilteil mit einer ähnlichen Öffnung (37) im Körper ausgebildet ist, wodurch das Ventil öffnet.

3. Hahn nach Anspruch 2, dadurch gekennzeichnet, daß der Ventilkörper eine luftdurchlässige Entlüftung (80, 82) der ähnlichen Öffnung des Körpers gegenüberliegend aufweist, wodurch, wenn das Ventil geöffnet ist, die luftdurchlässige Entlüftung geschlossen ist, und wenn das Ventil geschlossen ist, die luftdurchlässige Entlüftung geöffnet ist.

4. Hahn nach Anspruch 3, dadurch gekennzeichnet, daß die luftdurchlässige Entlüftung eine Öffnung (80) ist, die mit einem luftdurchlässigen Material (82) bedeckt ist.

5. Hahn nach Anspruch 4, dadurch gekennzeichnet, daß das luftdurchlässige Material mit einem Bakterizid imprägniert ist.

6. Hahn nach Anspruch 4, dadurch gekennzeichnet, daß das luftdurchlässige Material mit einem geruchtilgenden Material imprägniert ist.

7. Hahn nach Anspruch 1, dadurch gekennzeichnet, daß ein Handgriffsabschnitt (56) des Ventilteils sich im wesentlichen radial von ihm erstreckt.

8. Hahn nach Anspruch 7, dadurch gekennzeichnet, daß der Handgriff die Form einer flachen Lamelle aufweist, die an einem sich radial von dem Ventilteil nach außen erstreckenden Arm befestigt oder einstückig mit ihm ausgebildet ist.

9. Hahn nach Anspruch 8, dadurch gekennzeichnet, daß auf der einen Seite des Handgriffs eine Markierung oder Codierung (z.B. eine Farbe oder ein anderes Zeichen) zum Anzeigen, daß das Ventil "geschlossen" ist, vorgesehen ist.

10. Hahn nach Anspruch 8, dadurch gekennzeichnet, daß der Handgriff auf der anderen Seite eine Markierung oder Codierung aufweist, um anzuzeigen, daß das Ventil "geöffnet" ist.

11. Hahn nach einem der vorhergehenden Ansprüche, dadurch gekennzeichnet, daß der Körper einstückig mit einem flachen Flansch aus Kunststoff ausgebildet ist, der in einer gegenüberliegenden Weise mit der Wand des Sackes verschweißt ist.

12. Hahn nach einem der vorhergehenden Ansprüche in Kombination mit einem Sack, dadurch gekennzeichnet, daß der Körper einstückig mit einem Flansch (36) aus Kunststoff ausgebildet ist, und daß der Flansch mittels eines Klebstoffes in gegenüberliegender Weise mit einer Wand des Sackes (10) verklebt ist.

FIG.1

0 068 744

FIG. 2

FIG. 3

FIG. 4

FIG. 5

CLOSED

FIG.6

FIG.7